# EUROPÄISCHE PATENTSCHRIFT

(11) **EP 2 841 440 B1**
(45) Veröffentlichungstag und Bekanntmachung des Hinweises auf die Patenterteilung: **20.07.2016**
(21) Anmeldenummer: 13724527.0
(22) Anmeldetag: 25.04.2013
(51) Int. Cl.: C07F 15/00

(54) **VERFAHREN ZUR HERSTELLUNG VON DIKETONATO-RHODIUM(I)-CARBONYLKOMPLEXEN**
PROCESS FOR PREPARING DIKETONATO-RHODIUM(I)-CARBONYL COMPLEXES
PROCÉDÉ DE PRODUCTION DE COMPLEXES DICÉTONATE-RHODIUM(1)-CARBONYLE

(30) Priorität: 25.04.2012 DE 102012008111
(43) Veröffentlichungstag der Anmeldung: 04.03.2015
(73) Patentinhaber: Umicore AG & Co. KG, 63457 Hanau (DE)
(72) Erfinder: WOERNER, Eileen, 61130 Nidderau (DE); EBERT, Timo, 63796 Kahl (DE); KARCH, Ralf, 63801 Kleinostheim (DE); RIVAS-NASS, Andreas, 64625 Bensheim (DE); DOPPIU, Angelino, 63500 Seligenstadt (DE); WIDMER, Juergen, 63517 Rodenbach (DE)
(86) Internationale Anmeldenummer: PCT/EP2013/058628
(87) Internationale Veröffentlichungsnummer: WO 2013/160401

(56) Entgegenhaltungen:
- DU J ET AL: "The influence of precursors on Rh/SBA-15 catalysts for N2O decomposition", APPLIED CATALYSIS B: ENVIRONMENTAL, ELSEVIER, AMSTERDAM, NL, Bd. 84, Nr. 3-4, 1. Dezember 2008 (2008-12-01), Seiten 490-496, XP025562306, ISSN: 0926-3373, DOI: 10.1016/J.APCATB.2008.05.004 [gefunden am 2008-05-21]
- EWA MIECZYNSKA ET AL: "Hydroformylation and isomerization of hex-1-ene catalyzed by [Rh(acac)(CO)(PPh3)]: Effect of modifying ligands", JOURNAL OF MOLECULAR CATALYSIS, Bd. 73, Nr. 1, 1. April 1992 (1992-04-01), Seiten 1-8, XP055066856, ISSN: 0304-5102, DOI: 10.1016/0304-5102(92)80056-M
- POWELL AND B L SHAW J: "Transition metal-carbon bonds. Part XIII. Di-[mu]-chlorodicarbonyldi-(ethylene)dirho dium(I)", JOURNALS OF THE CHEMICAL SOCIETY. A, INORGANIC, PHYSICAL AND THEORETICAL CHEMISTRY, CHEMICAL SOCIETY, UK, 1. Januar 1968 (1968-01-01), Seiten 211-212, XP008163080, ISSN: 0022-4944, DOI: DOI: 10.1039/J19680000205

## Beschreibung

### Einleitung

Gegenstand der Erfindung ist ein Verfahren zur Herstellung von Diketonatorhodium(1)-carbonylkomplexen, insbesondere von Diketonato-carbonyltriorganophosphin-rhodium(I)-Komplexen wie beispielsweise Acetylacetonatocarbonyl-triphenylphosphin-rhodium(I) Rh(CO)(PPh₃)acac, hiernach auch als "Ropac" bezeichnet.

Das erfindungsgemäße Verfahren zeichnet sich durch eine verbesserte, einstufige Verfahrensdurchführung aus, die auf etwaige Zwischenisolierungs- oder Zwischenreinigungsstufen verzichtet (sogenannte "Eintopfsynthese"). In der vorliegenden Anmeldung wird ein Verfahren, das ggf. mehrere Verfahrensschritte umfasst, jedoch in einem einzigen Behältnis ohne Zwischenisolierungsschritte durchgeführt wird, auch als "einstufig" bezeichnet, was im Sinne der oben beschriebenen Eintopfsynthese zu verstehen ist.

Die vorliegende Erfindung ermöglicht durch das einstufige Verfahren eine schnelle Prozessführung bei der Herstellung der erfindungsgemäßen Diketonato-carbonyl-triorganophosphin-rhodium(I)-Komplexe. Durch die Verwendung kostengünstiger und umweltfreundlicher Lösungsmittel ist das Verfahren im industriellen Maßstab wirtschaftlich durchführbar. Ferner werden gute Ausbeuten erzielt.

Die hergestellten Diketonato-carbonyl-triorganophosphin-rhodium(I)-Komplexe sind besonders geeignet als Katalysatoren bzw. Präkatalysatoren für die Homogenkatalyse, wie beispielsweise für Hydroformylierungsreaktionen.

### Stand der Technik

Im Stand der Technik sind zahlreiche mehrstufige Verfahren zur Herstellung von Diketonato-carbonyl-triorganophosphin-rhodium(I)-Komplexen bekannt, die allesamt in dem Sinne mehrstufig sind, als Zwischenprodukte aufwändig isoliert werden müssen. In der Regel wird Rh(CO)(PPh₃)acac durch Umsetzung des Ausgangskomplexes Rh(I)-dicarbonyl-acetylacetonat Rh(CO)₂(acac) mit Triphenylphosphin hergestellt. Dabei wird Rh(CO)₂(acac) aus Rhodiumchlorid-Hydrat beispielsweise in Dimethylformamid unter Zusatz von Acetylaceton hergestellt, filtriert und gewaschen und anschließend umgesetzt.

Leipoldt et al. und Sheng-guo et al. beschreiben die Herstellung von Rh(CO)₂(acac) aus RhCl₃-Hydrat in Dimethylformamid/Acetylaceton durch Präzipitation nach Zusatz von Wasser, Abtrennung und Waschen der hergestellten Verbindung mit Petrolether (Siehe: J. G. Leipoldt, S. S. Basson, L. D. C. Bok und T. I. A. Gerber, Inorganica Chim Acta., 1978, 26, L35-L37; und: W. Sheng-guo, X. Xiao-dong, S. Guo-rong, Z. Ying-kui, Precious Metals, 2005, 26, 43-51 [Publikation in Chinesisch]).

In einem separaten Reaktionsschritt setzen Leipoldt et al. den hergestellten Rhodiumcarbonylkomplex in Benzol unter Zusatz von Triphenylphosphin (hiernach abgekürzt als PPh₃) zu Rh(CO)(PPh₃)acac um. Genaue Angaben zur Temperatur und Reaktionszeiten sind nicht offenbart, lediglich auf das Entfernen des Benzols nach Beendigung der reaktionsbedingten Kohlenmonoxidbildung wird verwiesen. Die Herstellung von Rh(CO)(PPh₃)acac erfolgt folglich mehrstufig. Angaben zur genauen Ausbeute fehlen.

Auch Sheng-guo et al. setzen das erhaltene Rh(CO)₂(acac) anschließend in einem separaten Reaktionsschritt unter Zusatz von PPh₃ in Toluol mit einer Reaktionszeit von einer Stunde zu Rh(CO)(PPh₃)acac um, wobei keine genauen Angaben zu den Reaktionstemperaturen enthalten sind. Mit dem beschriebenen mehrstufigen Prozess wird eine Ausbeute von 91 % erzielt.

Bonati und Wilkinson beschreiben in ihrer Synthese zunächst die Herstellung von Rh(CO)₂(acac) aus Tetracarbonyldichlorodirhodium [Rh(CO)₂Cl]₂ unter Zusatz von Bariumcarbonat und Acetylaceton in Petroleum durch etwa einwöchiges Rühren. Der hergestellte (CO)₂Rh(acac)-Komplex wird nach Abtrennen des Lösungsmittels in einer separaten Verfahrensstufe in Benzol unter Zusatz von PPh₃ zu Rh(CO)(PPh₃)acac umgesetzt. Auch Bonati und Wilkinson sehen daher ein mehrstufiges Verfahren vor. (Siehe: F. Bonati, G. Wilkinson, J. Chem. Soc., 1964, 3156-3160).

Auch Varshavsky et al. beschreiben ein Herstellverfahren ausgehend von [Rh(CO)₂Cl]₂. Rh(CO)(PPh₃)acac wird in einer separaten Verfahrensstufe aus Rhodiumcarbonylkomplexen unter Zusatz von PPh₃ in Hexan hergestellt. (Siehe: Y. S. Varshavsky, T. G. Cherkasova, N. A. Buzina, V. A. Kormer, J. Organomet. Chem., 1974. 77, 107-115).

Tic und Szymanowski beschreiben einen dreistufigen Prozess zur Herstellung von Rh(CO)(PPh₃)acac. Zunächst wird RhCl₃-Hydrat mit Kohlenmonoxid bei 70 bis 75°C für 0.5 bis 3.5 Stunden zur Reaktion gebracht. In einer zweiten Prozessstufe werden Bariumcarbonat und Acetylaceton zugesetzt bei Temperaturen von 45 bis 60°C für 0.25 bis 1,5 Stunden. Nachfolgend wird verbleibendes Bariumcarbonat und gebildetes Bariumchlorid durch Filtration sowie anschließend das Lösungsmittel durch Destillation entfernt, so dass das Verfahren zeitaufwändig ist. Die somit erhaltenen Kristalle des Zwischenproduktes Rh(CO)₂(acac) werden getrocknet. In einem nachgelagerten Prozessschritt wird Rh(CO)(PPh₃)acac durch Zusatz von PPh₃ in Hexan bei Temperaturen von 50°C über einen Zeitraum von 0.5 Stunden hergestellt. (Siehe: W. J. Tic, J. Szymanowski, Przemysl Chemiczny 2002, 81(6), 386-390 [Publikation in Polnisch]).

Es ist daher eine Aufgabe der vorliegenden Erfindung, ein Verfahren bereitzustellen, das eine kostengünstige, einstufige Herstellung von Diketonato-carbonyl-triorganophosphin-rhodium(I)-Komplexen, insbesondere von Rh(CO)(PPh₃)acac ("Ropac") im industriellen Maßstab erlaubt. Das Verfahren soll die Herstellung des Rhodiumcarbonylkomplexes zudem mit einer hohen Ausbeute sowie hoher Reinheit ermöglichen.

Die Aufgabe der vorliegenden Erfindung wird durch den Gegenstand der beigefügten Patentansprüche gelöst. Die erfindungsgemäße Lösung umfasst die Bereitstellung eines einstufigen Verfahren zur Herstellung von Diketonatocarbonyl-triorganophosphin-rhodium (I)-Komplexen, insbesondere von Rh(CO)(PPh₃)acac, welches aufgrund der eingesetzten Chemikalien, der Verfahrensführung und der erzielbaren hohen Ausbeuten zudem umweltfreundlich sowie wirtschaftlich ist.

Wie bereits erwähnt, umfasst ein einstufiges Herstellverfahren im Sinne der vorliegenden Erfindung die Herstellung der Diketonato-carbonyltriorganophosphin-rhodium (I)-Komplexe ohne jegliche Isolierung von Zwischenprodukten. Die vorliegende Erfindung beschreibt daher ein Verfahren, in dem das Zielprodukt ohne kosten- und zeitintensive Zwischenisolierungen oder Zwischenreinigungen in-situ in einem Reaktor aus den Ausgangsstoffen hergestellt wird (nachfolgend als "Eintopfsynthese" bezeichnet).

Das erfindungsgemäße Verfahren ermöglicht eine einfache Isolierung der Diketonato-carbonyl-triorganophosphin-rhodium(I)-Komplexe, da gezielt Verfahrensbedingungen gewählt werden, in denen die Zielverbindungen direkt aus dem Reaktionsgemisch ausfallen. Erfindungsgemäß umfasst dies die Bildung einer Suspension, welche die abtrennbaren Verbindungen als Niederschlag enthält. Somit ist keine zeit- und kostenaufwändige Isolierung beispielsweise durch Abtrennung, Aufkonzentrieren oder durch andere Prozesse erforderlich.

Die vorliegende Erfindung betrifft ein Verfahren zur Herstellung der Diketonato-carbonyl-triorganophosphin-rhodium(I)-Verbindung mit der allgemeinen Formel (I) 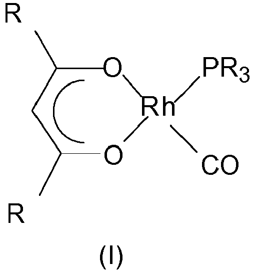 in der
- R: C₁-C₁₀-Alkyl-, C₅-C₁₀-Cycloalkyl-, C₆-C₁₂-Aryl-, C₄-C₁₂-Heteroaryl-Reste und
- R' und R": jeweils unabhängig voneinander C₁-C₅-Alkyl-, C₅-C₁₀-Cycloalkyl-, C₆-C₁₂-Aryl-Reste bedeuten,
wobei R, R' und R" gegebenenfalls substituiert sein können, umfassend die folgenden Reaktionsschritte:
(a) Einbringen eines Rh(III)-halogenid-Precursors in ein Lösungsmittel,
(b) Begasung mit Kohlenmonoxid (CO),
(c) Zugabe eines Diketons des Typs R'-C(=O)-CH₂-C(=O)-R" und einer Base, wobei die Zwischenverbindung (Diketonato)Rh(CO)₂ gebildet wird,
(d) Zugabe eines Triorganophosphins des Typs PR₃,
(e) Erhitzen des Reaktionsgemisches und Abtrennen des Diketonato-carbonyl-triorganophosphin-rhodium(I)-Komplexes der Formel (I), wobei als Lösungsmittel Alkohole, gegebenenfalls im Gemisch mit Wasser, als einheitliches Lösungsmittel über das gesamte Verfahren verwendet werden und das Verfahren einstufig ohne die Isolierung von Zwischenprodukten durchgeführt wird.

In einer bevorzugten Ausführungsform des Verfahrens wird der Diketonato-carbonyl-triorganophosphin-rhodium(I)-Komplex Rh(CO)(PPh₃)(acac) hergestellt. In diesem Falle kann das erfindungsgemäße Verfahren durch die folgenden idealisierten und beispielhaften Reaktionsgleichungen beschrieben werden:

Gleichung 1): RhCl₃ + 2 CO + CH₃CH₂₀H → ½ [Rh(CO)₂Cl]₂ + 2HCl + CH₃CHO

Gleichung 2): ½ [Rh(CO)₂Cl]₂ + Hacac + NaHCO₃ → [Rh(CO)₂(acac)] + NaCl + H₂O + CO₂

Gleichung 3): [Rh(CO)₂(acac)] + PPh₃ → [Rh(CO)(PPh₃)(acac)] + CO

Bei Gleichung 1) handelt es sich, wie bereits erwähnt, um eine idealisierte Reaktionsgleichung, die lediglich der Erläuterung dient. Es ist nicht bekannt, ob die Reduktion von Rh(III) durch CO oder Ethanol (oder ggf. durch beide) bewirkt wird.

Wesentlich ist jedoch für das erfindungsgemäße Verfahren, dass die Reaktion ausgehend von einer löslichen Rh(III)-Halogenidspezies in Gegenwart von CO und in Gegenwart eines organischen Lösungsmittels, bevorzugt in Gegenwart eines Alkohols und besonders bevorzugt in Gegenwart von Ethanol, erfolgt.

In der Reaktionsgleichung 2) wird eine Diketo-Verbindung, vorzugsweise eine 1,3-Diketo-Verbindung des Typs R'-C(=O)-CH₂-C(=O)-R" zugegeben. Eine 1,3-Diketo-Verbindung im Sinne der vorliegenden Erfindung ist ein organisches Molekül, das zwei Ketogruppen besitzt, die durch eine Methylengruppe voneinander getrennt sind.

Die Reste R' und R" können jeweils unabhängig voneinander C₁-C₅-Alkyl-, C₅-C₁₀-Cycloalkyl- oder C₆-C₁₂-Aryl-Reste bedeuten, wobei R' und R" gegebenenfalls substituiert sein können. Bevorzugt bedeuten die Reste R' und R" jeweils unabhängig voneinander C₁-C₅-Alkyl-, C₅-C₁₀-Cycloalkyl- oder C₆-C₁₂-Aryl-Reste, die nicht substituiert sind. Beispiele für geeignete Diketo-Verbindungen des Typs R'-C(=O)-CH₂-C(=O)-R" sind Acetylaceton (CH₃-CO-CH₂-CO-CH₃, R' = R" = Methyl), 2,4-Hexandion (R' = Methyl, R" = Ethyl, Trivialname Propionylaceton), 2,2-Dimethyl-3,5-hexandion (R' = Methyl, R" = *t*-butyl), 2,4-Heptandion (R' = Methyl, R" = Propyl; Trivialname Butanoylacteon), 6-Methyl-2,4-Heptandion (R' = methyl, R" = *iso*-propyl, Trivialname Isovalerylaceton) und 1,3-diphenyl-1,3-propandion (R' = R" = phenyl). Die besonders bevorzugte 1,3-Diketo-Verbindung ist Acetylaceton.

Im Laufe der Reaktion geht die 1,3-Diketo-Verbindung in die anionische Diketonato-Verbindung über und wird als zweizähniger, einfach negativ geladener Ligand an das Rh-Zentralatom gebunden, vgl. hierzu Reaktionsgleichung 2).

Zum Abfangen noch vorhandener und freigesetzter HCl und zur Sicherstellung einer vollständigen Reaktion setzt man eine Base (beispielsweise NaHCO₃) im Überschuss ein. Ein Vorteil beim Einsatz von Carbonaten oder Hydrogencarbonaten als Base ist die leichte Entfernung von Reaktionsprodukten (CO₂) über die Gasphase und damit die Verschiebung des Gleichgewichtes in Richtung des Produktes. Hierzu trägt auch die schlechte Löslichkeit der Reaktionsprodukte NaCl, wie auch des gebildeten (Diketonato)(CO)₂Rh in den gewählten Lösungsmitteln bei. Es resultiert eine Suspension.

Zum Reaktionsgemisch wird in Gleichung 3) eine Triorganophosphorverbindung zugesetzt. Dabei wird ein CO-Ligand substituiert. Es können Triorganophosphine des Typs PR₃, insbesondere Trialkylphosphine oder Triarylphosphine verwendet werden.

Im Triorganophosphin des Typs PR₃ bedeuten die Reste R im Allgemeinen C₁-C₁₀-Alkyl-, C₅-C₁₀-Cycloalkyl-, C₆-C₁₂-Aryl- oder C₄-C₁₂-Heteroaryl-Reste, die gegebenenfalls substituiert sein können. Beispiele sind Tributylphosphin, Triisobutylphosphin, Tricyclopentylphosphin, Tricyclohexylphosphin oder Tri(o-tolyl)-phosphin. Beispiele für Triorganophosphine mit Heteroarylresten sind Tri(2-furyl)phosphin oder Diphenyl(2-pyridyl)-phosphin.

Als Reste R sind C₁-C₁₀-Alkyl-, C₅-C₁₀-Cycloalkyl- oder C₆-C₁₂-Aryl-Reste bevorzugt. In einer besonders bevorzugten Ausführungsform handelt es sich um Triphenylphosphin (PPh₃).

Nachfolgend werden die einzelnen Reaktionsschritte a) bis e) des erfindungsgemäßen Verfahrens näher beschrieben.

### Reaktionsschritt a)

Einbringen eines zumindest teilweise löslichen Rh(III)-halogenid-Precursors in ein Lösungsmittel

Als Rh-halogenid-Precursor wird in der Regel Rhodium(III)-chlorid in Form einer Lösung, bzw. eines löslichen Feststoffs (z.B. RhCl₃xH₂O) eingesetzt, bevorzugt in Form einer gebrauchsfertigen wässrigen Rhodium(III)-chlorid-Lösung. Typischerweise werden wasserhaltigen Rhodium(III)-chlorid-Lösungen mit einem Rhodium-Gehalt < 30 Gew.-% verwendet, wie sie handelsüblich sind und beispielsweise durch Lösen von Rhodiummetall in Gegenwart von konzentrierter Salzsäure und Chlorgas hergestellt werden. Geeignete Rhodium(III)-chlorid-Lösungen können aber auch aus Prozessströmen des Edelmetallrecyclings oder der industriellen Edelmetallchemie abgezweigt werden. Darüber hinaus hat die Verwendung einer Rhodium(III)-chlorid-Lösung gegenüber dem üblicherweise verwendeten festen RhCl₃-Hydrat den Vorteil einer kostengünstigeren und schnelleren Verarbeitung, da ein vorgelagertes Eindampfen, Isolieren zu RhCl₃-Hydrat und Analysieren (zur Bestimmung der Ausgangsmenge) entfällt. Folglich ist gebrauchsfertige Rh(III)-Chlorid-Lösung als Edukt kurzfristiger verfügbar und einfacher handhabbar.

Der Rh(III)-Precursor wird in ein Lösungsmittel, vorzugsweise in ein organisches Lösungsmittel eingebracht. Liegt der Rh(III)-Precursor als wässrige Rhodium(III)-chlorid-Lösung vor, wird er mit dem Lösungsmittel gemischt. Liegt der Rh(III)-Precursor als festes Vorprodukt, beispielsweise RhCl₃-Hydrat vor, wird er im Lösungsmittel gelöst.

Bei dem Lösungsmittel handelt es sich vorzugsweise um niedrige aliphatische Alkohole wie Methanol, Ethanol oder Isopropanol. Gegebenenfalls können diese Alkohole mit Wasser gemischt werden. In einer bevorzugten Ausführungsform umfasst das Lösungsmittel Ethanol. In einer weiter bevorzugten Ausführungsform kann als Lösungsmittel die Diketo-Verbindung selbst, gegebenenfalls im Gemisch mit Wasser verwendet werden.

Die Verwendung von Alkoholen wie beispielsweise Ethanol ermöglicht den Einsatz eines einheitlichen Lösungsmittels über den gesamten Prozess, d.h. für die Herstellung von [(CO)₂RhCl]₂ (Gleichung (1)), als auch für (Diketona-to)Rh(CO)₂ (Gleichung (2)) und auch später für die Herstellung von (Diketonato)(PR₃)Rh(CO) (Gleichung (3)). Es ist damit keine Lösungsmittelabtrennung oder ein Lösungsmittelwechsel notwendig. Dadurch ergibt sich ein ökonomisch und ökologisch vorteilhaftes Verfahren.

Bevorzugte Lösungsmittel für das erfindungsgemäße Verfahren sind grundsätzlich solche, in denen sich der Rh(III)-Precursor und das Dicarbonylprodukt [(CO)₂RhCl]₂ gut lösen, während die Löslichkeit des Zwischenproduktes (Diketonato)(CO)₂Rh und insbesondere des Zielproduktes (Diketonato)(PR₃)Rh(CO) schlechter ist, so dass diese jeweils aus dem Reaktionsgemisch ausfallen und das Zielprodukt ohne aufwändige Konzentrationsschritte oder Lösungsmittelaustausch direkt durch Filtration isoliert werden kann. Desweiteren sollte auch das zum Einsatz kommende Triorganophosphin des Typs PR₃, vorzugsweise Triphenylphosphin (PPh₃), in diesem Lösungsmittel gut löslich sein.

Die Verwendung von Alkoholen wie Ethanol ermöglicht zudem eine kostengünstige und umweltschonendere Herstellung im Vergleich zu den für diesen Prozess im ersten Teilschritt zur Herstellung von (Diketonato)Rh(CO)₂ üblicherweise eingesetzten toxischen Lösungsmitteln wie Dimethylformamid (DMF).

### Reaktionsschritt b)

### Begasung mit Kohlenmonoxid (CO)

Die im Reaktionsschritt a) erhaltene Lösung wird nachfolgend zur Herstellung der Carbonylverbindung [Rh(CO)₂Cl]₂ mit Kohlenmonoxid (CO) versetzt. Es resultiert eine klare, gelbe Lösung an Rhodium(I)-Dicarbonylchlorid-Dimer. In einer speziellen Ausführungsform kann der Begasungsschritt bereits in Gegenwart der Diketo-Verbindung erfolgen (d.h. Reaktionsschritt c) kann vor Schritt b) erfolgen).

Die Begasung mit CO erfolgt bei erhöhten Reaktionstemperaturen im Bereich zwischen 25°C und 80°C. Bevorzugt werden die Reaktionstemperaturen zwischen 50°C und 70°C gewählt.

In einer bevorzugten Form läuft die Reaktion ohne Anwendung einer Druckatmosphäre ab. Die Durchführung der Reaktion in leichtem Unterdruck ist jedoch ebenfalls möglich.

Die Begasungszeit ist von den gewählten Temperatur- und Druckparametern abhängig und liegt typischerweise im Bereich von 2 bis 24 Stunden, bevorzugt 2 bis 16 Stunden, um eine vollständige Umsetzung zu erreichen. Während der CO-Begasung sollte eine intensive Verteilung des CO in der Lösung sichergestellt sein. Die feine Verteilung des CO in der Lösung kann durch schnelles Rühren während der Einleitung sichergestellt werden, alternativ können Begasungsrührer oder Gasverteiler eingesetzt werden.

### Reaktionsschritt c)

Zugabe der Diketo-Verbindung (bevorzugt Acetylaceton) und einer Base wobei das Zwischenprodukt (Diketonato)Rh(CO)₂ gebildet wird

Das in Schritt b) erhaltene Reaktionsgemisch wird ohne Zwischenisolierung des in-situ erhaltenen Zwischenproduktes [Rh(CO)₂Cl]₂ auf Temperaturen unter 40°C, bevorzugt unter 35°C abgekühlt. Anschließend erfolgt die Zugabe der Diketo-Verbindung und einer Base. Eine Base ist erfindungsgemäß eine Verbindung, die den pH-Wert einer wässrigen Lösung auf ph ≥7, erfindungsgemäß bevorzugt auf einen pH-Wert im Bereich zwischen pH 7 und 10, weiter bevorzugt auf pH 7 bis 8 erhöhen bzw. einstellen kann.

Erfindungsgemäß bevorzugt wird zuerst die Diketo-Verbindung sowie anschließend die Base zugesetzt.

Die Base ist generell ausgewählt aus der Gruppe der Alkalicarbonate, Erdalkalicarbonate, Alkalihydrogencarbonate, Erdalkalihydrogencarbonate, Alkalidiketonate, Erdalkalidiketonate und Gemischen und Kombinationen davon. Beispiele für geeignete anorganische Basen sind NaHCO₃, Na₂CO₃, K₂CO₃, CaCO₃ und BaCO₃. Beispiele für geeignete organische Basen sind Alkali- oder Erdalkalidiketonate wie beispielsweise Na(acac) und Ca(acac)₂. Idealerweise wird in diesem Fall die Diketo-Verbindung verwendet, welche auch im Zielprodukt als Diketonat-Ligand vorliegt. Die erfindungsgemäß bevorzugte Base ist NaHCO₃.

Bei der Verwendung kommerzieller Rhodium(III)-chloridlösung, ist die Zugabe von 2 bis 12 Moläquivalenten an Base (z.B. NaHCO₃) bezogen auf die eingesetzte Rh-Menge) vorteilhaft; bevorzugt werden etwa 5 bis 10 Moläquivalente an Base (bezogen auf die eingesetzte Rh-Menge) zur ausreichenden Neutralisation der in der Rhodiumchloridlösung enthaltenen sauren Komponenten eingesetzt.

Es zeigt sich, dass die Diketo-Verbindung mindestens in einer Menge von 2 Moläquivalenten, bevorzugt in einer Menge von 5 bis 12 Moläquivalenten und besonders bevorzugt in einer Menge von 8 bis 12 Moläquivalenten (bezogen auf die eingesetzte Rh-Menge) zugesetzt werden sollte. Optional kann nach Zugabe der Base die Reaktionsmischung bei Temperaturen unter 40°C nachgerührt werden. Das Zwischenprodukt (Diketonato)Rh(CO)₂, insbesondere Rh(CO)₂(acac) fällt bei dieser Reaktion in der Regel aus, so dass ein Reaktionsgemisch erhalten wird, in dem das Zwischenprodukt (Diketonato)Rh(CO)₂ suspendiert (d.h. in einer Suspension) vorliegt.

### Reaktionsschritt d)

Zugabe eines Triorganophosphins zum Reaktionsgemisch

Das nach dem oben beschriebenen Verfahren erzeugte Zwischenprodukt (Diketonato)Rh(CO)₂ wird weiter zum Zielprodukt (Diketonato)Rh(CO)(PR₃) umgesetzt, das in der Regel in Form eines gelben Feststoffs anfällt. Es zeigt sich überraschenderweise, dass eine Zwischenisolierung von (Diketonato)Rh(CO)₂ zur Herstellung von (Diketonato)Rh(CO)(PR₃) nicht erforderlich ist. Erfindungsgemäß erfolgt daher die Darstellung von (Diketonato)Rh(CO)(PR₃)acac direkt aus dem im Reaktionsschritt c) erhaltenen Reaktionsgemisch durch Zusatz einer Triorganophosphorverbindung (bevorzugt Triphenylphosphin, PPh₃).

Es ist festzuhalten, dass diese Reaktion aus einer Suspension zu einer weiteren Suspension führt. (Diketonato)Rh(CO)₂ weist eine geringe Restlöslichkeit im gewählten Lösungsmittel auf. Das restgelöste Material reagiert mit (teilweise) gelöstem Phosphin PR₃; dabei fällt das Endprodukt aus der Reaktionsmischung aus, daneben wird CO freigesetzt. In der Regel ist die Löslichkeit des Endproduktes (Diketonato)(PR₃)(CO)Rh in Alkoholen geringer als die des Zwischenproduktes (Diketonato)(CO)₂Rh.

Das Triorganophosphin des Typs PR₃ wird dabei in einem Anteil von 0.98 bis 1.08 Moläquivalenten bezogen auf Rh hinzugefügt. Erfindungsgemäß bevorzugt werden 1.01 bis 1.03 Moläquivalente Phosphin zugesetzt, da dies eine hohe Ausbeute an reiner Zielverbindung sicherstellt. Weiterhin wird durch diese Maßnahme die Zielverbindung in höherer Reinheit erhalten, da nur sehr geringe Mengen an chlorhaltigem Nebenprodukt gebildet werden.

Erfindungsgemäß bevorzugt wird dabei das Triorganophosphin in Reinform, im Falle von PPh₃ als Feststoff zugesetzt. Die Zugabe von gelöstem Phosphin ist ebenfalls möglich. In diesem Fall werden organische Lösungsmittel verwendet, in denen sich das Triorganophosphin löst wie in Anspruch 1 definiert.

Die Zugabe erfolgt in der Regel bei einer Temperatur über 15°C und bis zu 40°C, wobei in der Regel die Reaktionsmischung gerührt wird.

### Reaktionsschritt e)

Erhitzen des Reaktionsgemisches, anschließendes Abkühlen und Abtrennen des Rh(I)-Zielproduktes

Nach Zugabe des Triorganophosphins wird das Reaktionsgemisch unter Rückfluss erhitzt ("refluxiert"), wobei je nach verwendetem Lösungsmittel bzw. Lösungsmittelgemisch Temperaturen im Bereich von 50 bis 120°C eingestellt werden. Die maximale Erhitzungstemperatur orientiert sich am gewählten Lösungsmittel bzw. Lösungsmittelgemisch. Die Refluxzeit für eine vollständige Umsetzung sollte in der Regel bei 1 bis 20 Stunden, vorzugsweise 2 bis 16 Stunden liegen. Dieser Reaktionsschritt ist nicht in allen Fällen notwendig, allerdings ermöglicht er die Isolierung von sehr reinem Produkt.

Nach (optionalem) Abkühlen kann die Verbindung vom Reaktionsgemisch abgetrennt werden, bevorzugt durch Filtration. Das Waschen erfolgt in der Regel unter Verwendung wassermischbarer organischer Lösungsmittel und/oder vollentsalztem Wasser (VE-Wasser). Durch eine Waschung mit VE-Wasser lässt sich das Endprodukt chloridfrei waschen. Zur Entfernung weiterer Verunreinigungen werden weiterhin aliphatische Alkohole (vorzugsweise Ethanol) eingesetzt. Das Produkt wird üblicherweise bei Temperaturen im Bereich von 30 bis 60°C, vorzugsweise im Bereich von 35 bis 50°C getrocknet. Die Trocknung erfolgt bevorzugt im Vakuum, kann jedoch auch unter Luft oder Inertgas (Argon, Stickstoff etc) erfolgen.

Bei Einhaltung der Bedingungen und Schritte des oben dargestellten erfindungsgemäßen einstufigen Herstellverfahrens können die Zielprodukte des Typs (Diketonato)(PR₃)(CO)Rh, insbesondere Rh(CO)(PPh₃)acac in hoher Ausbeute und Reinheit erhalten werden. Für das erfindungsgemäße Verfahren liegen die Ausbeuten über 93%, bevorzugt über 95%. Hierbei wird die Ausbeute auf die enthaltenen Rhodiumgehalte bezogen, d.h. die Ausbeute "OMB" ("on metal base") zugrunde gelegt. Insgesamt wird mit dem Verfahren der vorliegenden Erfindung die Prozessdauer üblicher mehrstufiger Herstellverfahren von Rh(CO)(PPh₃)acac um mehr als die Hälfte reduziert. Dies führt zu einer hohen wirtschaftlichen Bedeutung des Verfahrens.

Aufgrund der guten Ausbeute und der hohen Reinheit des Endproduktes sowie aufgrund der kosteneffizienten und umweltfreundlichen Parameterwahl des Verfahrens ist es für den industriellen Maßstab prädestiniert.

Der mit dem erfindungsgemäßen Eintopfverfahren hergestellte Rhodiumcarbonylkomplex Rh(CO)(PPh₃)acac ("Ropac") eignet sich aufgrund der hervorragenden Reinheit sowie der resultierenden hohen katalytischen Aktivität besonders als Katalysator bzw. Präkatalysator, bevorzugt bei Hydroformylierungsreaktionen beispielsweise zur Umsetzung von Alkenen. Die in dieser Reaktion hergestellten Alkohole sind vielseitig industriell einsetzbar.

### Analytische Daten

Die erfindungsgemäß hergestellten Rh-Verbindungen des Typs (Diketonato)(PR₃)(CO)Rh, insbesondere Rh(CO)(PPh₃)acac ("Ropac") zeichnen sich durch einen niedrigen Gehalt an Chlor (Gesamt-Chlor-Gehalt, incl. freies und gebundenes Chlorid nach Wickboldt) aus. Der Anteil an chloridischen Verunreinigungen liegt in der Regel bei < 0.1 Gew.-% (1.000 ppm), bevorzugt bei < 0.03% (300 ppm), ermittelt mittels eines Chloranalyzers (Fa. Analytik Jena). Aufgrund dieser niedrigen Chlorwerte ist die gute katalytische Aktivität der erfindungsgemäß hergestellten Verbindung sichergestellt.

Im ³¹P-NMR-Spektrum des Produktes Rh(CO)(PPh₃)acac ("Ropac") kann insbesondere der Anteil der Verbindung Rh(CO)(PPh₃)₂Cl festgestellt werden (z.B. durch Signalaufspaltung bei etwa 30 ppm). In der Regel beträgt der prozentuale Flächenanteil des Peaks von Rh(CO)(PPh₃)₂Cl im ³¹P-NMR-Spektrum bezogen auf den Flächenanteil des Peaks von Rh(CO)(PPh₃)acac weniger als 5 %, bevorzugt weniger als 1 %, besonders bevorzugt weniger als 0.3 % (gemessen in CDCl₃ und bezogen auf die Fläche der peaks beider Verbindungen im ³¹P-NMR-Spektrum). Die Verbindung Rh(CO)(PPh₃)₂Cl weist nur geringe katalytische Aktivität auf und sollte daher nur in sehr geringen Mengen im Produkt Rh(CO)(PPh₃)acac zugegen sein.

Das erfindungsgemäße Verfahren wird anhand der folgenden Beispiele näher erläutert.

### Beispiel 1

In einem 10 I - Doppelmantelreaktor mit Rührer, Strömungsbrecher, Rückflusskühler und einem Gaseinleitrohr, welches mit einer Glasfritte ausgestattet ist, werden 1.6 kg Ethanol (technische Qualität) unter Rühren vorgelegt. Unter weiterem Rühren werden 200.0 g Rhodium (1.94 mol) in Form von Rhodium(III)-chlorid-Lösung zur Lösung gegeben (Rh(III)-chlorid-Lösung, Umicore-Produkt-Nr. 68.2565.2720; Rh-Gehalt ca. 20 Gew.-%)

Das Vorratsgebinde der Rhodiumchloridlösung wird mehrfach mit insgesamt 1.7 kg Ethanol nachgewaschen und dies zur Lösung im Reaktor hinzugefügt. Der Reaktor wird auf 25°C temperiert und über das Gaseinleitrohr wird Kohlenmonoxid (CO; Qualität 2.0, Fa. Linde) durch die Reaktionsmischung bei einem Gasstrom von ca. 65 l/h geführt. Man erhitzt zügig auf eine Reaktorinnentemperatur von ca. 60°C. Ab Erreichen der Begasungstemperatur von 60°C wird für ca. 7 h die Begasung fortgesetzt. Danach wird zügig auf eine Reaktorinnentemperatur von 30°C abgekühlt und anschließend die Gaszufuhr beendet.

Unter Rühren werden zur Reaktionsmischung 2.14 kg Acetylaceton (zur Synthese) gegeben. Innerhalt von 30 Minuten werden portionsweise 1.63 kg Natriumhydrogencarbonat (reinst) über einen Feststofftrichter dosiert. Hierbei schäumt die Reaktionsmischung durch das freigesetzte Kohlendioxid auf. Nach vollständiger Dosierung wird weitere 60 Minuten bei 30°C gerührt. Zur resultierenden Suspension werden innerhalb von 20 Minuten 515 g Triphenylphosphin (1.96 mol, min. 99.5%, Imhoff & Stahl) dosiert. Hierbei wird Kohlenmonoxid freigesetzt. Nach ca. 30 minütigem Rühren wird die Reaktionsmischung für ca. 16 h bei ca. 74°C Innentemperatur unter Rückfluss erhitzt. Nach Abkühlen auf Raumtemperatur wird die Suspension über eine geeignete Glasfritte filtriert. Der Filterkuchen wird mehrfach mit insgesamt 0.8 kg Ethanol gewaschen und anschließend in mehreren Portionen mit VE-Wasser chloridfrei gewaschen (Cl-Bestimmung anhand der Restleitfähigkeit, sowie durch Fällungsprüfung mit Silbernitratlösung). Nach erneutem Waschen mit 0,8 kg Ethanol und 0.7 kg Petrolether (50/70) wird der Filterkuchen im Vakuumtrockenschrank bei 40°C getrocknet.

Man erhält ca. 953.5 g gelben Feststoff mit einem Rhodiumgehalt von 20.76 %. (Bestimmung des Rh-Gehaltes per ICP-OES nach erfolgtem Aufschluss mit Schwefelsäure und Salpetersäure). Dies entspricht einer rhodiumbasierten Ausbeute von 99 %. Der Gesamtchlorgehalt liegt bei < 0.03 % (Wickboldt-Aufschluss).

Die Identität des Produktes (PPh₃)(CO)Rh(acac) wird über ³¹P-NMR-Spektroskopie bestimmt (in CDCl₃). Man erhält ein Dublett bei ca. 50 ppm (Signale bei 49.5 und 51.1 ppm). Der prozentuale Flächenanteil des Peaks des Nebenproduktes Rh(CO)(PPh₃)₂Cl im ³¹P-NMR-Spektrum ist bezogen auf den Flächenanteil des Peaks von Rh(CO)(PPh₃)acac < 1%.

Eine Wiederholung des Ansatzes mit einer CO-Begasung über 16 h ergibt eine rhodiumbasierte Ausbeute von 99.7 %, einen Cl-Gehalt von < 0.03 %.

**Referenzbeispiel 2** 40.0 g Rhodium (0.39 mol) in Form von 101.9 g Rh(III)chlorid-hydrat (Umicore-Produktnr. 68.2562.1138, Gehalt 39.25 % Rh), 437 ml Acetylaceton (4,29 mol) und 431 ml Ethanol (techn. Qualität) werden in einem 1 l - Doppelmantelreaktor mit Rückflusskühler, KPG-Rührer und Gaseinleitrohr vorgelegt. Die Mischung wird mit einem CO-Gasstrom von 20 l/h unter Rühren begast und hierbei auf 60°C erwärmt. Unter diesen Bedingungen wird 13 h gehalten und anschließend auf ca. 30°C abgekühlt. Die CO Begasung wird abgestellt. Zur Lösung werden in kleinen Portionen über einen Zeitraum von ca. 30 Minuten 326.5 g NaHCO₃ dosiert. Hierbei wird CO₂ freigesetzt. Es wird 1 Stunde gerührt. Anschließend werden 105.0 g (0,4 mol) Triphenylphosphin in 400 ml Toluol gelöst und unter starkem Rühren innerhalb von 20 Minuten zur Reaktionsmischung zugefügt. Direkt anschließend (ohne Zwischenrührzeit) wird die Reaktionsmischung auf Refluxtemperatur (ca. 73°C) erwärmt. Nach 8 h Refluxieren wird auf 25°C abgekühlt und der resultierende Feststoff über eine Glasfritte abfiltriert und portionsweise mit 15 Litern VE-Wasser chloridfrei gewaschen. Der Feststoff wird anschließend im Vakuum bei 40°C bis zur Gewichtskonstanz getrocknet. Es resultieren 38.04 g Rhodium in Form von 182.0 g (PPh₃)(CO)Rh(acac) mit einem Rh-Gehalt von 20.9 %, entsprechend einer rhodiumbasierten Ausbeute von 95.1 %. Es wird ein Gesamt-Cl-Gehalt < 0.03 %. Die Identität und Reinheit des Produktes (PPh₃)(CO)Rh(acac) wird mittels ³¹P-NMR-Spektroskopie (in CDCl₃) bestätigt.

## Patentansprüche

1. Verfahren zur Herstellung des Diketonato-carbonyl-triorganophosphinrhodium(I)-Komplexes der Formel (I) 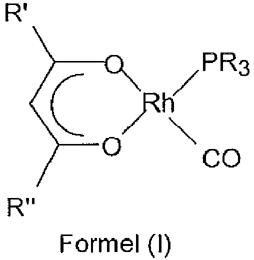 in der
R C₁-C₁₀-Alkyl, C₅-C₁₀-Cycloalkyl, C₆-C₁₂-Aryl oder C₄-C₁₂-Heteroaryl und
R', R" jeweils unabhängig voneinander C₁-C₅-Alkyl, C₅-C₁₀-Cycloalkyl oder C₆-C₁₂-Aryl bedeuten, wobei R, R' und R" optional substituiert sein können,
umfassend die Reaktionsschritte:
(a) Einbringen eines Rh(III)-halogenid-Precursors in ein Lösungsmittel,
(b) Begasung mit Kohlenmonoxid (CO),
(c) Zugabe einer Diketo-Verbindung des Typs R'-C(=O)-CH₂-C(=O)-R" und einer Base, wobei die Zwischenverbindung (Diketonato)Rh(CO)₂ gebildet wird,
(d) Zugabe eines Triorganophosphins des Typs PR₃,
(e) Erhitzen des Reaktionsgemisches und Abtrennen des Diketonatocarbonyl-triorganophosphin-rhodium(I)-Komplexes der Formel (I), wobei als Lösungsmittel Alkohole, gegebenenfalls im Gemisch mit Wasser, als einheitliches Lösungsmittel über das gesamte Verfahren verwendet werden und das Verfahren einstufig ohne die Isolierung von Zwischenprodukten durchgeführt wird.

2. Verfahren nach Anspruch 1, wobei als Diketo-Verbindung Acetyl-aceton, 2,4-Hexandion, 2,2-Dimethyl-3,5-hexandion, 2,4-Heptandion, 6-Methyl-2,4-Heptandion oder 1,3-Diphenyl-1,3-propandion, vorzugsweise Acetylaceton eingesetzt wird.

3. Verfahren nach Anspruch 1 oder 2, wobei als Rh(III)-halogenid-Precursor im Reaktionsschritt a) eine wasserhaltige Rhodium(III)-chloridlösung oder festes Rh(III)Cl₃-Hydrat verwendet wird.

4. Verfahren nach einem der Ansprüche 1 bis 3, wobei als Triorganophosphin des Typs PR₃ Triphenylphosphin, Tributylphosphin, Triisobutylphosphin, Tricyclopentylphosphin, Tricyclohexylphosphin oder Tri-(o-tolyl)-phosphin, vorzugsweise Triphenylphosphin, eingesetzt wird.

5. Verfahren nach einem der Ansprüche 1 bis 4, wobei als Lösungsmittel im Reaktionsschritt a) ein niedriger aliphatischer Alkohol aus der Gruppe Methanol, Ethanol oder Isopropanol oder Gemische davon verwendet werden.

6. Verfahren nach einem der Ansprüche 1 bis 5, wobei als Lösungsmittel im Reaktionsschritt a) Ethanol, gegebenenfalls in einer Mischung mit Wasser, eingesetzt wird.

7. Verfahren nach einem der Ansprüche 1 bis 6, wobei die in Schritt c) gebildete Zwischenverbindung Diketonato-Rh(C4)₂ im Reaktionsgemisch in Suspension vorliegt.

8. Verfahren nach einem der Ansprüche 1 bis 7, wobei die Begasung mit CO im Reaktionsschritt b) bei Temperaturen zwischen 25 und 80°C, vorzugsweise bei Temperaturen zwischen 50°C und 70°C erfolgt.

9. Verfahren nach einem der Ansprüche 1 bis 8, wobei die Begasung mit CO über einen Zeitraum von 2 bis 24 Stunden, vorzugsweise über einen Zeitraum von 2 bis 16 Stunden erfolgt.

10. Verfahren nach einem der Ansprüche 1 bis 9, wobei als Base im Reaktionsschritt c) eine anorganische Base aus der Gruppe NaHCO₃, Na₂CO₃, K₂CO₃, CaCO₃ und BaCO₃ oder eine organische Base aus der Gruppe der Alkali- oder Erdalkali-Diketonate eingesetzt wird.

11. Verfahren nach einem der Ansprüche 1 bis 10, wobei die Diketo-Verbindung in Schritt c) in einer Menge von 2 bis 12 Moläquivalenten, vorzugsweise in einer Menge von 5 bis 10 Moläquivalenten pro Mol Rh zugesetzt wird.

12. Verfahren nach einem der Ansprüche 1 bis 11, wobei im Reaktionsschritt d) das Triorganophosphin in einer Menge von 0.98 bis 1.08 Mol- äquivalenten, bevorzugt in einer Menge von 1.01 bis 1.03 Moläquivalenten pro Mol Rh zugesetzt wird.

13. Verfahren nach einem der Ansprüche 1 bis 12, wobei die Zugabe des Triorganophosphins im Reaktionsschritt d) bei einer Temperatur von 15 bis 40°C erfolgt.

14. Verfahren nach einem der Ansprüche 1 bis 13, wobei das Erhitzen im Reaktionsschritt d) bei Temperaturen im Bereich von 50 bis 120°C über einen Zeitraum von 1 bis 20 Stunden, vorzugsweise über einen Zeitraum von 2 bis 16 Sunden erfolgt.

15. Verfahren nach einem der Ansprüche 1 bis 14, wobei das Verfahren eine Eintopfsynthese darstellt.

16. Verfahren nach einem der Ansprüche 1 bis 15, weiterhin umfassend das Abtrennen des Diketonato-carbonyl-triorganophosphin-rhodium(I)-Komplexes vom Reaktionsgemisch.

17. Verfahren nach Anspruch 16, weiterhin umfassend das Waschen des Diketonato-carbonyl-triorganophosphin-rhodium(I)-Komplexes unter Verwendung von wassermischbaren organischen Lösungsmitteln.

18. Verfahren nach einem der Ansprüche 1 bis 17, wobei der Diketonato-carbonyl-triorganophosphin-rhodium(I)-Komplex die Formel (II) besitzt:

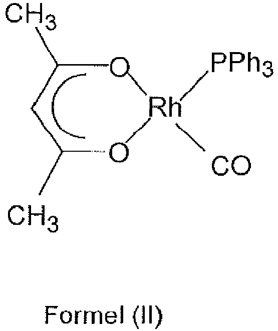

19. Verfahren zur Homogenkatalyse von chemischen Reaktionen, insbesondere Hydroformylierungsreaktionen, durch Verwendung des Komplexes Rh(CO)(PPh₃)acac als Katalysator oder Präkatalysator mit den Schritten
- Bereitstellen des Komplexes Rh(CO)(PPh₃)acac nach einem oder mehreren der Ansprüche 1 bis 18;
- Durchführen der chemischen Reaktionen unter Verwendung des so erhaltenen Rh(CO)(PPh₃)acac als als Katalysator oder Präkatalysator.

## Claims

1. A process for preparing the diketonatocarbonyltriorganophosphinerhodium(I) complex having the formula (I) 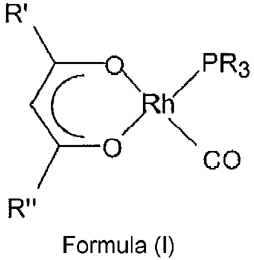 where
R is C₁-C₁₀-alkyl, C₅-C₁₀-cycloalkyl, C₆-C₁₂-aryl, or C₄-C₁₂-heteroaryl and
R', R" " are each, independently of one another, a C₁-C₅-alkyl, C₅-C₁₀-cycloalkyl, or C₆-C₁₂-aryl, where R, R', and R" may optionally be substituted,
comprising the reaction steps:
(a) introduction of an Rh(III) halide precursor into a solvent,
(b) treatment with carbon monoxide (CO) gas,
(c) addition of a diketo compound of the type R'-C(=O)-CH₂-C(=O)-R" and a base, forming the intermediate (diketonato)Rh(CO)₂,
(d) addition of a triorganophosphine of the type PR₃,
(e) heating of the reaction mixture and isolation of the diketonatocarbonyltriorganophosphinerhodium(I) complex having the formula (I), wherein, as solvent, alcohols - optionally mixed with water - are used as uniform solvent throughout the entire process, and the process is carried out in a single stage, without isolation of intermediates.

2. A process according to claim 1, wherein acetylacetone, 2,4-hexanedione, 2,2-dimethyl-3,5-hexanedione, 2,4-heptanedione, 6-methyl-2,4-heptanedione, or 1,3-diphenyl-1,3-propanedione - preferably, acetylacetone - are used as a diketo compound.

3. A process according to claim 1 or 2, wherein a water-containing rhodium(III) chloride solution or solid Rh(III)Cl₃ hydrate is used as Rh(III) halide precursor in reaction step a).

4. A process according to one of claims 1 through 3, wherein triphenylphosphine, tributylphosphine, triisobutylphosphine, tricyclopentylphosphine, tricyclohexylphosphine, or tri(o-tolyl)-phosphine - preferably, triphenylphosphine - is used as triorganophosphine of the type PR₃.

5. A process according to one of claims 1 through 4, wherein a lower aliphatic alcohol from the group consisting of methanol, ethanol, or isopropanol, or mixtures thereof, is used as solvent in reaction step a).

6. A process according to one of claims 1 through 5, wherein ethanol - optionally, in a mixture with water - is used as solvent in reaction step a).

7. A process according to one of claims 1 through 6, wherein the intermediate diketonato-Rh(CO)₂ formed in step c) is present in suspension in the reaction mixture.

8. A process according to one of claims 1 through 7, wherein the treatment with CO gas in reaction step b) is carried out at temperatures between 25 °C and 80 °C - preferably, at temperatures between 50 °C and 70 °C.

9. A process according to one of claims 1 through 8, wherein the treatment with CO gas is carried out over a period of from 2 to 24 hours - preferably, over a period of from 2 to 16 hours.

10. A process according to one of claims 1 through 9, wherein an inorganic base selected from the group consisting of NaHCO₃, Na₂CO₃, K₂CO₃, CaCO₃, and BaCO₃ or an organic base selected from the group consisting of alkali or alkaline earth diketonates is used as base in reaction step c).

11. A process according to one of claims 1 through 10, wherein the diketo compound is added in an amount of from 2 to 12 molar equivalents - preferably, in an amount of from 5 to 10 molar equivalents - per mole of Rh in step c).

12. A process according to one of claims 1 through 11, wherein the triorganophosphine is added in an amount of from 0.98 to 1.08 molar equivalents - preferably, in an amount of from 1.01 to 1.03 molar equivalents - per mole of Rh in reaction step d).

13. A process according to one of claims 1 through 12, wherein the addition of the triorganophosphine in reaction step d) is carried out at a temperature of from 15 °C to 40 °C.

14. A process according to one of claims 1 through 13, wherein the heating in reaction step d) is carried out at temperatures in the range from 50 °C to 120 °C over a period of from 1 to 20 hours - preferably, over a period of from 2 to 16 hours.

15. A process according to one of claims 1 through 14, wherein the process is a one-pot synthesis.

16. A process according to one of claims 1 through 15, comprising further the separation of the diketonatocarbonyltriorganophosphinerhodium(I) complex from the reaction mixture.

17. A process according to claim 16, comprising further the washing of the diketonatocarbonyltriorganophosphinerhodium(I) complex using water-miscible organic solvents.

18. A process according to one of claims 1 through 17, wherein the diketonatocarbonyltriorganophosphinerhodium(I) complex has the formula (II):

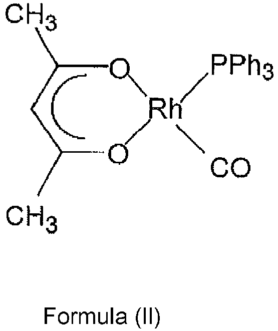

19. A process for homogeneous catalysis of chemical reactions - in particular, hydroformylation reactions - through use of the Rh(CO)(PPh₃)acac complex as catalyst or precatalyst with the steps
- providing the Rh(CO)(PPh₃)acac complex according to one or more of claims 1 through 18;
- executing the chemical reactions using the Rh(CO)(PPh₃)acac thus obtained as catalyst or precatalyst.

## Revendications

1. Procédé de fabrication du complexe de dicétonato-carbonyl-triorganophosphine-rhodium(I) de formule (I) 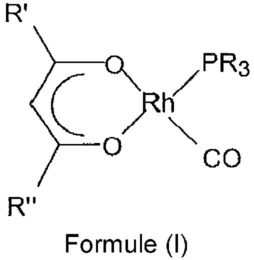 dans laquelle
R signifie alkyle en C₁-C₁₀, cycloalkyle en C₅-C₁₀, aryle en C₆-C₁₂ ou hétéroaryle en C₄-C₁₂ et
R', R" signifient chacun indépendamment l'un de l'autre alkyle en C₁-C₅, cycloalkyle en C₅-C₁₀ ou aryle en C₆-C₁₂, R, R' et R" pouvant éventuellement être substitués,
comprenant les étapes de réaction :
(a) introduction d'un précurseur d'halogénure de Rh(III) dans un solvant,
(b) injection de monoxyde de carbone (CO),
(c) ajout d'un composé dicéto de type R'-C(=O)-CH₂-C(=O)-R" et d'une base, le composé intermédiaire (dicétonato)Rh(CO)₂ étant formé,
(d) ajout d'une triorganophosphine de type PR₃,
(e) chauffage du mélange réactionnel et séparation du complexe de dicétonato-carbonyl-triorganophosphine-rhodium(I) de formule (I), dans lequel des alcools, éventuellement en mélange avec de l'eau, sont utilisés en tant que solvant sous la forme d'un solvant uniforme dans l'ensemble du procédé et le procédé est réalisé en une étape sans l'isolement de produits intermédiaires.

2. Procédé selon la revendication 1, dans lequel l'acétylacétone, la 2,4-hexanedione, la 2,2-diméthyl-3,5-hexanedione, la 2,4-heptanedione, la 6-méthyl-2,4-heptanedione ou la 1,3-diphényl-1,3-propanedione, de préférence l'acétylacétone, est utilisée en tant que composé dicéto.

3. Procédé selon la revendication 1 ou 2, dans lequel une solution de chlorure de rhodium(III) contenant de l'eau ou un hydrate de Rh(III)Cl₃ solide est utilisé dans l'étape de réaction a) en tant que précurseur d'halogénure de Rh(III).

4. Procédé selon l'une quelconque des revendications 1 à 3, dans lequel la triphénylphosphine, la tributylphosphine, la triiso-butylphosphine, la tricyclopentylphosphine, la tricyclohexylphosphine ou la tri-(o-tolyl)-phosphine, de préférence la triphénylphosphine, est utilisée en tant que triorganophosphine de type PR₃.

5. Procédé selon l'une quelconque des revendications 1 à 4, dans lequel un alcool aliphatique inférieur du groupe constitué par le méthanol, l'éthanol ou l'isopropanol ou leurs mélanges est utilisé en tant que solvant dans l'étape de réaction a).

6. Procédé selon l'une quelconque des revendications 1 à 5, dans lequel de l'éthanol, éventuellement en mélange avec de l'eau, est utilisé en tant que solvant dans l'étape de réaction a).

7. Procédé selon l'une quelconque des revendications 1 à 6, dans lequel le composé intermédiaire dicétonato-Rh(CO)₂ formé à l'étape c) se présente en suspension dans le mélange réactionnel.

8. Procédé selon l'une quelconque des revendications 1 à 7, dans lequel l'injection de CO dans l'étape de réaction b) a lieu à des températures comprises entre 25 et 80 °C, de préférence à des températures comprises entre 50 °C et 70 °C.

9. Procédé selon l'une quelconque des revendications 1 à 8, dans lequel l'injection de CO a lieu pendant une période de 2 à 24 heures, de préférence pendant une période de 2 à 16 heures.

10. Procédé selon l'une quelconque des revendications 1 à 9, dans lequel une base inorganique du groupe constitué par NaHCO₃, Na₂CO₃, K₂CO₃, CaCO₃ et BaCO₃ ou une base organique du groupe constitué par les dicétonates alcalins ou alcalino-terreux est utilisée en tant que base dans l'étape de réaction c).

11. Procédé selon l'une quelconque des revendications 1 à 10, dans lequel le composé dicéto est ajouté dans l'étape c) en une quantité de 2 à 12 équivalents molaires, de préférence en une quantité de 5 à 10 équivalents molaires par mole de Rh.

12. Procédé selon l'une quelconque des revendications 1 à 11, dans lequel la triorganophosphine est ajoutée dans l'étape de réaction d) en une quantité de 0,98 à 1,08 équivalent molaire, de préférence en une quantité de 1,01 à 1,03 équivalent molaire par mole de Rh.

13. Procédé selon l'une quelconque des revendications 1 à 12, dans lequel l'ajout de la triorganophosphine dans l'étape de réaction d) a lieu à une température de 15 à 40 °C.

14. Procédé selon l'une quelconque des revendications 1 à 13, dans lequel le chauffage dans l'étape de réaction d) a lieu à des températures dans la plage allant de 50 à 120 °C, pendant une période de 1 à 20 heures, de préférence pendant une période de 2 à 16 heures.

15. Procédé selon l'une quelconque des revendications 1 à 14, dans lequel le procédé est une synthèse monotope.

16. Procédé selon l'une quelconque des revendications 1 à 15, comprenant en outre la séparation du complexe de dicétonato-carbonyl-triorganophosphine-rhodium(I) du mélange réactionnel.

17. Procédé selon la revendication 16, comprenant en outre le lavage du complexe de dicétonato-carbonyl-triorganophosphine-rhodium(I) en utilisant des solvants organiques miscibles avec l'eau.

18. Procédé selon l'une quelconque des revendications 1 à 17, dans lequel le complexe de dicétonato-carbonyl-triorganophosphine-rhodium(I) présente la formule (II) :

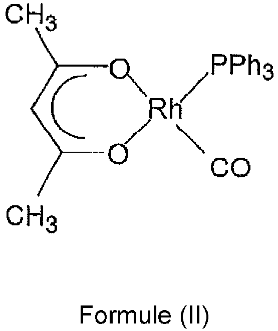

19. Procédé de catalyse homogène de réactions chimiques, notamment de réactions d'hydroformylation, utilisant le complexe Rh(CO)(PPh₃)acac en tant que catalyseur ou pré-catalyseur, comprenant les étapes :
- préparation du complexe Rh(CO)(PPh₃)acac selon une ou plusieurs des revendications 1 à 18 ;
- la réalisation des réactions chimiques en utilisant le Rh(CO)(PPh₃)acac ainsi obtenu en tant que catalyseur ou pré-catalyseur.
